# EUROPÄISCHE PATENTSCHRIFT

(11) **EP 1 021 241 B1**
(45) Veröffentlichungstag und Bekanntmachung des Hinweises auf die Patenterteilung: **29.08.2001**
(21) Anmeldenummer: 97910417.1
(22) Anmeldetag: 06.10.1997
(51) Int. Cl.: B01J 2/04, B01J 2/00, A61K 9/00

(54) **VERFAHREN ZUR HERSTELLUNG EINES PULVERFÖRMIGEN PRODUKTES AUS EINEM FLÜSSIGEN STOFF ODER STOFFGEMISCH**
METHOD FOR PRODUCING A POWDER PRODUCT FROM A LIQUID SUBSTANCE OR MIXTURE OF SUBSTANCES
PROCEDE DE PRODUCTION D'UN PRODUIT SOUS FORME DE POUDRE A PARTIR D'UNE SUBSTANCE LIQUIDE OU D'UN MELANGE DE SUBSTANCES LIQUIDE

(43) Veröffentlichungstag der Anmeldung: 26.07.2000
(73) Patentinhaber: Adalbert-Raps-Stiftung, 95326 Kulmbach (DE)
(72) Erfinder: WEINREICH, Bernd, D-82327 Tutzing (DE); STEINER, Rudolf, D-91056 Erlangen (DE); WEIDNER, Eckhard, D-91056 Erlangen (DE); DIRSCHERL, Johann, D-95326 Kulmbach (DE)
(74) Vertreter: Beyer, Andreas, Dr.
(86) Internationale Anmeldenummer: EP9705484
(87) Internationale Veröffentlichungsnummer: WO9917868

(56) Entgegenhaltungen:
- WO-A-95/21688
- WO-A-96/15133
- WO-A-98/16204

## Beschreibung

Die Erfindung betrifft ein Verfahren zur Herstellung eines pulverförmigen Produktes aus einem flüssigen Stoff oder Stoffgemisch, wie es im Oberbegriff des Patentanspruchs 1 angegeben ist. Ein solches Verfahren ist aus der WO 95/21688 bekannt.

Pulverförmige Produkte werden häufig aufgrund ihrer im Vergleich zu Flüssigkeiten einfacheren Handhabung bevorzugt. Im Normalfall ist beispielsweise der Transport und die Lagerung eines pulverförmigen Produkts gegenüber einer Flüssigkeit unkritischer. Zur Herstellung von Pulvern sind mechanische Verfahren, wie beispielsweise Mahlen und Agglomerieren, und thermische Verfahren, wie beispielsweise Kristallisation und Sprühtrocknung, bekannt. Stoffe, die mit solchen klassischen Verfahren pulverisiert werden, weisen in der Regel einen Schmelzpunkt auf, der deutlich oberhalb der Umgebungstemperatur (Raumtemperatur) liegt. Das bedeutet, daß der Aggregatzustand dieser Stoffe durch die Pulverisierung nicht geändert wird.

Stoffe, deren Schmelzpunkt unterhalb der normalen Umgebungstemperatur liegt, können erst pulverisiert werden, wenn sie durch Kühlen zum Erstarren gebracht worden sind. Auch nach dem Pulverisieren kann der feste Aggregatzustand solcher Stoffe nur im Rahmen aufwendiger Kühlketten beibehalten werden. Eine andere Möglichkeit Stoffe zu stabilisieren, die bei normaler Umgebungstemperatur flüssig sind, besteht darin, die zu stabilisierende Substanz auf feinteilige Trägerpartikel aufzubringen. Dabei werden die Trägerpartikel mit Hilfe eines Gasstromes fluidisiert und die zu stabilisierende, flüssige Substanz wird auf die fluidisierten Trägerpartikel aufgesprüht. Mittels dieses Verfahrens, das als Wirbelschichtcoating bekanntgeworden ist, werden die Trägerpartikel mit einem dünnen Film der zu stabilisierenden, flüssigen Substanz überzogen. Das Massenverhältnis zwischen dem Träger und der solchermaßen stabilisierten Substanz wird durch die Abmessungen und die Gestalt der Trägerpartikel sowie von der Beschichtungsdicke bestimmt. Die erreichbaren Wirkstoffkonzentrationen (unter Wirkstoff wird hier die zu stabilisierende, bei Raumtemperatur flüssige Substanz verstanden) liegen zwischen 1 Gew.% und höchstens etwa 10 Gew.% bezogen auf die fertig beschichteten Trägerpartikel. Hinzu kommt, daß eine solche Beschichtungstechnik nur bei bestimmten Materialkombinationen angewandt werden kann, bei denen die gegenseitigen Benetzungs- und Haftkräfte die Erzeugung eines beschichteten Trägerpartikels erlauben. Eine weitere Einschränkung ergibt sich aus der zu stabilisierenden Flüssigkeit: Ist deren Viskosität zu hoch, kann sie nicht oder nur mit großem Aufwand versprüht werden. Bei manchen Flüssigkeiten läßt sich die Versprühbarkeit durch eine Verdünnung mit geeigneten Lösungsmitteln verbessern, jedoch bedeutet dies zusätzlichen Aufwand und reduziert im übrigen den Wirkstoffgehalt im fertig stabilisierten Produkt, ganz abgesehen von der Tatsache, daß die Verwendung vieler Lösungsmittel heutzutage unter physiologischen und Umweltgesichtspunkten unerwünscht ist.

Das bereits erwähnte, aus der WO 95/21688 bekannte Verfahren ermöglicht es, aus Flüssigkeiten pulverförmige Feststoffe herzustellen. Das Verfahrensprinzip besteht darin, in der zu pulverisierenden Flüssigkeit unter erhöhtem Druck ein Gas zu lösen, vorzugsweise bis zum Erhalt einer gasgesättigten Lösung. Im Vergleich zur reinen Flüssigkeit hat eine solche Lösung eine Reihe von günstigen Eigenschaften: So ist normalerweise die Viskosität dieser Lösung gegenüber der reinen Flüssigkeit bei gleicher Temperatur um mehrere Größenordnungen erniedrigt und auch die Grenzflächenspannung ist deutlich verringert. Die unter Druck stehende Flüssigkeits/Gas-Lösung wird anschließend einem Entspannungsorgan zugeleitet und dort schnell entspannt. Dabei kühlt sich das in der Flüssigkeits/Gas-Lösung enthaltene Gas unter starker Volumenzunahme deutlich ab und trennt sich von der Flüssigkeit. Dieser Vorgang führt zur Entstehung kleiner Feststoffpartikel, die ausschließlich aus der vorher flüssigen Substanz bestehen. Die entstandenen Feststoffpartikel können mit üblichen Verfahren abgetrennt und, falls gewünscht, fraktioniert werden.

Obwohl dieses bekannte Verfahren eine elegante und einfache Methode darstellt, Flüssigkeiten in ein pulverförmiges Produkt umzuwandeln, muß nach wie vor das erhaltene, pulverförmige Produkt gekühlt werden, wenn der Schmelzpunkt der verarbeiteten Substanz unterhalb der normalen Umgebungstemperatur liegt.

Der Erfindung liegt daher die Aufgabe zugrunde, ein Verfahren bereitzustellen, mit dem bei Raumtemperatur bzw. bei Umgebungstemperatur flüssige Stoffe oder Stoffgemische in Pulverform stabilisiert werden können, wobei die erhaltene Pulverform bei Raumtemperatur bzw. normaler Umgebungstemperatur beständig sein soll.

Diese Aufgabe ist ausgehend vom eingangs genannten Verfahren erfindungsgemäß dadurch gelöst, daß dem zu pulverisierenden, flüssigen Stoff oder Stoffgemisch bzw. der Flüssigkeits/Gas-Lösung vor dem Entspannungsorgan, in dem Entspannungsorgan oder nach, insbesondere kurz nach dem Entspannungsorgan ein fester, pulverförmiger Hilfsstoff zugemischt wird. In dem erfindungsgemäßen Verfahren reichen überraschenderweise bereits relativ geringe Hilfsstoffzugaben dazu aus, das beim schnellen Entspannen der Flüssigkeits/Gas-Lösung entstehende, pulverförmige Produkt zu stabilisieren. Dabei wird umso weniger fester Hilfsstoff benötigt, je höher die Schmelztemperatur der zu pulverisierenden Ausgangssubstanz ist. So können mit dem erfindungsgemäßen Verfahren puiverförmige Produkte hergestellt werden, die einen hohen Wirkstoffgehalt aufweisen. Das bedeutet, daß bei vielen Stoffen oder Stoffgemischen eine vergleichsweise geringere Hilfsstoffmenge, beispielsweise 1 bis 90 Gew.%, bevorzugt 10 bis 80 Gew.% und besonders bevorzugt nur 20 bis 50 Gew.% zur Stabilisierung der erhaltenen Pulverform ausreichen. Derart hohe Wirkstoffkonzentrationen konnten mit den bisher bekannten Verfahren nicht erreicht werden.

Bei dem Entspannen der Flüssigkeits/Gas-Lösung kann die Erstarrungstemperatur des Stoffes oder Stoffgemisches unterschritten werden, jedoch ist dies nicht unbedingt erforderlich, um das gewünschte, pulverförmige Produkt zu erhalten. Es hat sich bei einer Reihe von Anwendungsfällen allerdings als günstig herausgestellt, beim Entspannen der Flüssigkeits/Gas-Lösung eine Temperatur zu erreichen, die zumindest in die Nähe der Erstarrungstemperatur des Stoffes oder Stoffgemisches liegt.

Als Gas kann im Prinzip jedes Gas verwendet werden, das sich ausreichend in dem zu pulverisierenden, flüssigen Stoff oder Stoffgemisch löst. Beispielsweise können als Gas Kohlendioxid, ein Kohlenwasserstoff, insbesondere Methan, Ethan, Propan, Butan, Ethen, Propen oder ein halogenierter Kohlenwasserstoff, ein Ether, ein Inertgas, insbesondere Stickstoff, Helium oder Argon, ein gasförmiges Oxid, insbesondere Distickstoffoxid oder Schwefeldioxid, und Ammoniak verwendet werden. Auch kann eine Mischung aus zwei oder mehreren der vorgenannten Gase verwendet werden.

Der erhöhte Druck, unter dem das Gas in dem flüssigen Stoff oder Stoffgemisch aufgelöst wird, kann im Bereich von 5 bar bis 800 bar liegen, vorzugsweise liegt der Druck jedoch im Bereich von 10 bar bis 350 bar und besonders bevorzugt im Bereich von 20 bar bis 250 bar.

Vorzugsweise wird das Auflösen des Gases in dem flüssigen Stoff oder Stoffgemisch durch Vermischen des Gases mit dem flüssigen Stoff oder Stoffgemisch beschleunigt. Dieses Vermischen kann beispielsweise durch ein Schütteln oder Rollen des Druckbehälters erreicht werden, in dem die zu pulverisierende Flüssigkeit vorgelegt ist. Alternativ kann die im Druckbehälter entstehende Lösung mittels eines Rührers gerührt werden. Eine noch andere Möglichkeit, eine gute Durchmischung der zu pulverisierenden Flüssigkeit mit dem Gas zu erreichen, besteht darin, die im Druckbehälter enthaltene Flüssigphase und/oder die Gasphase zu rezirkulieren, d.h. aus dem Druckbehälter abzupumpen und dem Druckbehälter im Bereich der jeweils anderen Phase wieder zuzuführen. Eine noch andere Möglichkeit ist die Verwendung eines statischen Mischers. Selbstverständlich lassen sich die vorgenannten Verfahrensweisen auch kombinieren.

Das erfindungsgemäße Verfahren funktioniert prinzipiell mit jedem festen, pulverförmigen Hilfsstoff. Besonders geeignet sind allerdings Hilfsstoffe mit möglichst kleiner Partikelgröße, weshalb gemäß einer bevorzugten Ausgestaltung des erfindungsgemäßen Verfahrens die Partikelgröße kleiner als 100 *µ*m und insbesondere kleiner als 50 *µ*m ist. Hilfsstoffe, die eine poröse innere Struktur aufweisen, deren innere Oberfläche also möglichst groß ist, sind besonders gut zur Verwendung im erfindungsgemäßen Verfahren geeignet. Als Beispiele für Stoffe mit großer innerer Oberfläche seien hier Zeolithe und Aktivkohle genannt. Allgemein erfolgt die Auswahl eines geeigneten Hilfsstoffes nach technischen, physiologischen und gegebenenfalls auch nach lebensmittelrechtlichen Aspekten. Ohne Anspruch auf Vollständigkeit seien hier als mögliche Hilfsstoffe Stärke, modifizierte Stärke, Kochsalz, Zucker, Eiweiße, Gelatine, Titandioxid, Magnesiumstearat, Polyglykole, hochdisperses Siliciumdioxid, Kieselsäure, Bentonit, Kalk, Glutamat, Emulgatoren, insbesondere Phospholipide oder Partialglyceride, Fette, Cellulose und Cellulosederivate, Polymilchsäure, Wachse, Dextrin, Kaolin, Verdickungsmittel, insbesondere Alginate oder Pectin, feinstvermahlene Pflanzenteile oder auch eine Mischung aus zwei oder mehreren der vorgenannten Stoffe, die jeweils pulverförmig vorliegen müssen, genannt.

Je nach dem zu pulverisierenden, flüssigen Stoff oder Stoffgemisch werden sich bestimmte Hilfsstoffe zur Verwendung im erfindungsgemäßen Verfahren anbieten: Beispielsweise sind Phospholipide als Hilfsstoff zur Stabilisierung der gewünschten Pulverform grundsätzlich gut geeignet. Phospholipide stellen darüber hinaus natürliche, hochwirksame Emulgatoren sowohl für Wasser-in-Öl-Emulsionen als auch für Öl-in-Wasser-Emulsionen dar. Phospholipide verbessern demnach die Wasserdispergierbarkeit von öllöslichen Substanzen und die Öldispergierbarkeit von wasserlöslichen Substanzen. Sie werden also insbesondere dann als Hilfsstoff eingesetzt werden, wenn es auf diese zusätzlichen Eigenschaften ankommt. Polyethylenglykole, hochdisperses Siliciumdioxid, Stärke, modifizierte Stärke und Magnesiumstearat sind wasserlöslich oder gut dispergierbar und stellen Lösungsvermittler für nicht wasserlösliche Substanzen dar. Die Verwendung dieser Substanzen als Hilfsstoffe im erfindungsgemäßen Verfahren stabilisiert daher nicht nur wie gewünscht die Pulverform, sondern verbessert gleichzeitig die Wasserdispergierbarkeit bzw. Wasserlöslichkeit öllöslicher Stoffe oder Stoffgemische.

Beim erfindungsgemäßen Verfahren soll die Hilfsstoffkonzentration bezogen auf die Gesamtmenge an flüssigem Stoff oder Stoffgemisch und Hilfsstoff so niedrig wie möglich sein. Besonders bevorzugt beträgt die Hilfsstoffkonzentration deshalb nur bis zu 25 Gew.%. Sollten Hilfsstoffkonzentrationen bis 25 Gew.% nicht dazu ausreichen, den ehemals flüssigen, pulverisierten Stoff zu stabilisieren, können bevorzugt auch bis zu 50 Gew.% Hilfsstoffkonzentration im erfindungsgemäßen Verfahren eingesetzt werden. Bei manchen zu pulverisierenden, flüssigen Stoffen oder Stoffgemischen kann es erforderlich sein, die Hilfsstoffkonzentration noch höher zu wählen, etwa bis 90 Gew.%. Selbst diese relativ hohe Hilfsstoffkonzentration führt aber immer noch zu Wirkstoffgehalten, die deutlich über den Wirkstoffgehalten liegen, die mit herkömmlichen Verfahren erreichbar sind.

Als Entspannungsorgan kann im erfindungsgemäßen Verfahren jede Vorrichtung verwendet werden, die eine ausreichend schnelle Entspannung der Flüssigkeits/Gas-Lösung ermöglicht. Bevorzugt wird als Entspannungsorgan eine Düse, ein Diffusor, eine Kapillare, eine Blende, ein Ventil oder eine Kombination der vorgenannten Entspannungsorgane verwendet.

Es ist bei dem erfindungsgemäßen Verfahren wichtig, daß der zugegebene feste, pulverförmige Hilfsstoff mit der Flüssigkeits/Gas-Lösung bzw. - je nachdem wo die Zuführung des pulverförmigen Hilfsstoffs erfolgt - mit dem zu pulverisierenden Stoff oder Stoffgemisch vermischt wird. Bei ungenügender Durchmischung kann es dazu kommen, daß ein Teil des erhaltenen pulverförmigen Produkts nicht ausreichend stabilisiert ist und später bei Raumtemperatur bzw. bei normaler Umgebungstemperatur schmilzt oder anschmilzt.

Um eine gute Durchmischung des Hilfsstoffes mit der Flüssigkeits/Gas-Lösung bzw. dem zu pulverisierenden Stoff oder Stoffgemisch zu erreichen, stehen verschiedene Möglichkeiten zur Verfügung. So kann der pulverförmige Hilfsstoff beispielsweise dort zudosiert werden, wo die Flüssigkeits/Gas-Lösung aus dem Entspannungsorgan austritt, also an oder kurz vor der Entspannungsstelle. Der Hilfsstoff wird dann in dem sich nach der Entspannungsstelle bildenden Freistrahl mitgerissen, wobei die starke und schnelle Volumenexpansion des in der Flüssigkeits/Gas-Lösung enthaltenen Gases für eine äußerst intensive Verwirbelung und Vermischung des Hilfsstoffes mit dem zu pulverisierenden Stoff oder Stoffgemisch sorgt.

Gemäß einer anderen Ausführungsform des erfindungsgemäßen Verfahrens wird der Hilfsstoff derart zugeführt, daß er den aus dem Entspannungsorgan austretenden Stoffstrom im Bereich der Austrittsstelle ringförmig umgibt. Mit anderen Worten: Der Hilfsstoff wird - beispielsweise mit einer ringförmigen Düse - um den aus dem Entspannungsorgan austretenden Freistrahl zugegeben, so daß der Freistrahl jedenfalls anfänglich vom Hilfsstoff umhüllt ist. Die beim Austritt des Freistrahles aus dem Entspannungsorgan auftretenden Turbulenzen sorgen für eine gute Durchmischung des Hilfsstoffes mit dem feinen Spray des zu pulverisierenden Stoffes oder Stoffgemisches. Die Umhüllung des Freistrahles mit dem Hilfsstoff sorgt zudem dafür, daß kurz nach dem Austritt aus dem Entspannungsorgan eventuell noch vorhandene Flüssigkeitströpfchen sich nicht an einer umgebenden Wandung ablagern können, sondern mitgerissen werden. Gemäß einer Weiterbildung des erfindungsgemäßen Verfahrens werden der aus dem Entspannungsorgan austretende Stoffstrom und der Hilfsstoff in eine Art Diffusor geführt, mit dem die Aufweitung des Freistrahles kontrolliert werden kann. Der Diffusor kann zusätzlich eine oder mehrere Strömungsabrißkanten aufweisen, um mit der dort entstehenden Verwirbelung eine noch intensivere Durchmischung zwischen dem Freistrahl und dem Hilfsstoff zu bewirken.

Bei bevorzugten Ausgestaltungen des erfindungsgemäßen Verfahrens erfolgt die Entspannung der Flüssigkeits/Gas-Lösung in einen Sprühturm. Der zuzumischende Hilfsstoff kann dann beispielsweise mittels pneumatischer Förderung in den Sprühturm transportiert und an der gewünschten Stelle zudosiert werden. Alternativ kann das kalte Pulver aus dem Sprühturm entnommen und in einem separaten Mischer mit vorgekühltem, pulverförmigem Hilfsstoff bei einer Temperatur unterhalb des Schmelzpunktes des zu pulverisierenden Stoffes oder Stoffgemisches gemischt werden.

Bei bevorzugten Ausführungsformen des erfindungsgemäßen Verfahrens wird zusätzlich zu dem Gas, das bereits in dem zu pulverisierenden Stoff oder Stoffgemisch gelöst ist, weiteres Gas im Bereich des Entspannungsorgans zugegeben, das als sogenanntes Überschußgas bezeichnet werden kann. Mit diesem Überschußgas läßt sich die beim Entspannungsvorgang erreichte Temperatur unabhängiger einstellen. Weder ist es erforderlich, daß die Flüssigkeits/Gas-Lösung im wesentlichen mit dem Gas gesättigt ist, noch muß beispielsweise ein relativ hoher Druck gewählt werden, um eine zur gewünschten Abkühlung ausreichend große Gaskonzentration in der Flüssigkeit zu erreichen. Vielmehr kann die gewünschte Abkühlung im Bereich der Entspannungsstelle weitgehend durch die schnelle Entspannung des zusätzlich zugeführten Überschußgases hervorgerufen werden. Darüber hinaus ergibt sich die Möglichkeit, als Überschußgas ein Gas zu wählen, das verschieden von dem in der Flüssigkeit aufgelösten Gas ist. Beispielsweise kann das Überschußgas im Hinblick auf eine möglichst große Temperaturabsenkung ausgewählt werden, während das in der Flüssigkeit aufzulösende Gas nach anderen Gesichtspunkten festgelegt wird. Neben einer besseren Abkühlung im Bereich der Entspannungsstelle führt das Überschußgas auch zu einer noch besseren Durchmischung bzw. Verwirbelung nach dem Austritt des Stoffstroms aus dem Entspannungsorgan und damit zu noch kleineren Pulverpartikeln.

Hinsichtlich der Zuführung des Überschußgases bestehen verschiedene Möglichkeiten. Gemäß einer Ausgestaltung des erfindungsgemäßen Verfahrens wird das Überschußgas zwischen dem Druckbehälter und dem Entspannungsorgan, insbesondere kurz vor der Entspannungsstelle, in die Flüssigkeits/Gas-Lösung eingespeist. Hierbei kann zur besseren Vermischung mit der Flüssigkeits/Gas-Lösung beispielsweise ein statischer Mischer eingesetzt werden.

Gemäß einer anderen Ausgestaltung werden in dem Entspannungsorgan mittels einer Zweistoffdüse die Flüssigkeits/Gas-Lösung und zusätzlich zugeführtes Überschußgas gemeinsam miteinander entspannt. Bei dieser Ausgestaltung wird das Überschußgas also nicht der Flüssigkeits/Gas-Lösung zugegeben, sondern direkt an der Entspannungsstelle zugeführt, so daß die Flüssigkeits/Gas-Lösung und das reine Überschußgas gleichzeitig entspannt werden. Die Zweistoffdüse kann beispielsweise derart sein, daß die Flüssigkeits/Gas-Lösung durch einen zentralen Kanal austritt, während das Überschußgas durch einen Ringkanal austritt, der den zentralen Kanal koaxial umgibt.

Gemäß einer noch anderen Ausgestaltung wird das Überschußgas zusammen mit dem festen, pulverförmigen Hilfsstoff der Lösung bzw. dem Stoff oder Stoffgemisch zugeführt.

Wenn vorliegend von zu pulverisierendem Stoff oder Stoffgemisch die Rede ist, dann bedeutet dies, daß die zu pulverisierende Flüssigkeit kein Reinstoff sein muß, sondern daß es sich dabei durchaus um eine Mischung oder Lösung verschiedener Flüssigkeiten oder Stoffe handeln kann, wobei die Flüssigkeiten bzw. Stoffe sowohl organische als auch anorganische Flüssigkeiten bzw. Stoffe sein können. Des weiteren kann einem flüssigen Reinstoff ein weiterer Stoff zugegeben sein, der die Eigenschaften des entstehenden pulverförmigen Endprodukts in gewünschter Weise beeinflußt. Beispielsweise kann einer zu pulverisierenden, wasserunlöslichen Flüssigkeit ein Emulgator zugegeben werden, um so eine verbesserte Wasserdispergierbarkeit des pulverförmigen Endprodukts zu erreichen. Der zu pulverisierende, flüssige Stoff bzw. das Stoffgemisch kann auch eine Suspension sein.

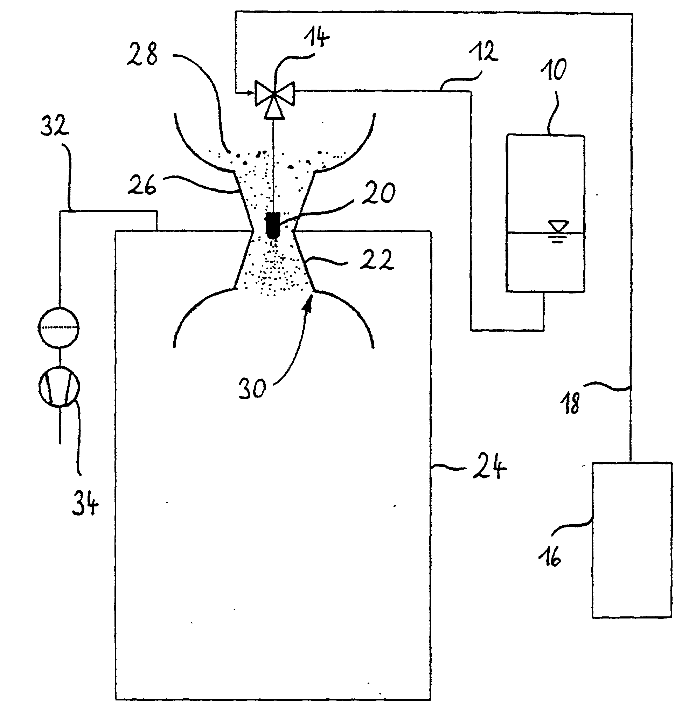

Im folgenden wird anhand der einzigen Figur eine Apparatur näher erläutert, die mit Vorteil zur Durchführung des erfindungsgemäßen Verfahrens verwendet werden kann.

Die Figur zeigt als Druckbehälter einen Autoklav 10, in den der zu pulverisierende, flüssige Stoff oder das Stoffgemisch eingefüllt werden. Anschließend wird durch geeignete Maßnahmen, beispielsweise durch Bewegen des Autoklaven 10 oder des Autoklaveninhaltes, ein ausgewähltes Gas in der vorgelegten Flüssigkeit unter Druck aufgelöst. Die Zuführung des ausgewählten Gases erfolgt auf herkömmliche Weise und ist in der Figur nicht dargestellt. Zur Beschleunigung des Auflösens von Gas in der zu pulverisierenden Flüssigkeit können die Flüssigkeit und das darin aufzulösende Gas im Gleichstrom durch einen statischen Mischer geführt und anschließend in den Autoklaven 10 eingeleitet werden. Je nach Art des ausgewählten Gases und abhängig von dem gewählten Druck sowie der Temperatur können in der flüssigen Phase Gaskonzentrationen zwischen 1 und 90 Gew.%, vorzugsweise 5 bis 50 Gew.% und insbesondere 10 bis 40 Gew.% erreicht werden. Die Temperatur liegt zweckmäßigerweise im Bereich der Raum- bzw. Umgebungstemperatur, jedoch kann bei hochviskosen Stoffen oder Stoffgemischen auch eine höhere Temperatur erforderlich sein. Wesentlich ist, daß der zu pulverisierende Stoff bzw. das Stoffgemisch im Druckbehälter als Flüssigkeit oder Suspension vorliegt.

Die nach dem Auflösen des Gases im Autoklaven 10 vorhandene Flüssigkeits/Gas-Lösung wird über eine Leitung 12 einem Dreiwegeventil 14 zugeführt. Aus einem Gasbehälter 16 wird über eine Leitung 18 zusätzliches Gas, sogenanntes Überschußgas, dem Dreiwegeventil 14 zugeführt. Das Überschußgas kann ein anderes als das in der Flüssigkeit aufgelöste Gas sein.

Aus dem Dreiwegeventil 14 werden die Flüssigkeits/Gas-Lösung sowie das zugeführte Überschußgas einem Entspannungsorgan zugeleitet, das hier eine Hochdruckdüse 20 ist. Zwischen der Hochdruckdüse 20 und dem Dreiwegeventil 14 kann ein weiterer statischer Mischer vorgesehen sein, um die Einmischung des Überschußgases in die Flüssigkeits/Gas-Lösung zu verbessern.

Die Hochdruckdüse 20 ist an der engsten Stelle eines Diffusors 22 angeordnet, der im Deckel eines Sprühturmes 24 befestigt ist. Über einen mit dem Diffusor 22 verbundenen Trichter 26 wird ein fester, pulverförmiger Hilfsstoff 28 kontinuierlich zugegeben, solange die Flüssigkeits/Gas-Lösung und das Überschußgas aus der Hochdruckdüse 20 ausströmen. Zwischen der Hochdruckdüse 20 und der Innenwand des Trichters 26 bzw. des Diffusors 22 ist ein sich zunächst verengender, dann wieder erweiternder Ringspalt gebildet, durch den der zugegebene Hilfsstoff 28 strömt. Der Hilfsstoff umgibt also den aus der Hochdruckdüse 20 ausströmenden Stoffstrom ringförmig. Die Förderung des Hilfsstoffes 28 in den Trichter 26 kann mittels bekannter Methoden erfolgen, beispielsweise mittels pneumatischer Förderung, durch Rüttelschienen, mittels eines Schneckendosierers, einer Zellradschleuse oder ähnlichem.

Die starke Volumenzunahme des in der Flüssigkeits/Gas-Lösung enthaltenen Gases sowie des zusätzlich zugeführten Überschußgases nach dem Austritt aus der Hochdruckdüse 20 führt zu einer starken Turbulenz und damit zu einer guten Durchmischung des Hilfsstoffs mit dem aus der Hochdruckdüse 20 austretenden Stoffstrom. Im gezeigten Ausführungsbeispiel erhöht eine im Diffusor vorhandene Strömungsabrißkante 30 die Turbulenz noch.

Die starke Abkühlung, die mit der Entspannung des in der Flüssigkeit gelösten Gases sowie des Überschußgases einhergeht, sorgt zusammen mit der erwähnten, hohen Turbulenz für eine so schnelle und intensive Durchmischung mit dem Hilfsstoff, daß bereits bei einer Sprühturmhöhe von nur 1 m das gewünschte pulverförmige Endprodukt erhalten wird. Das Pulver sammelt sich im unteren Teil des Sprühturms 24 und kann auf herkömmliche Art entnommen werden.

Das in der Flüssigkeit gelöste Gas sowie das Überschußgas trennen sich nach dem Austritt aus der Hochdruckdüse 20 von dem zu pulverisierenden Stoff oder Stoffgemisch. Im gezeigten Ausführungsbeispiel wird das solchermaßen freigesetzte Gas im oberen Bereich des Sprühturms 24 durch eine Leitung 32 abgezogen. Durch die zwischen dem Diffusor 22 und der Sprühturminnenwand vorhandene Beruhigungszone wird ein Austrag feiner Partikel durch die Leitung 32 vermieden. Ein gegebenenfalls dennoch in dem abgesaugten Gas enthaltener Feinanteil pulverisierten Produkts kann auf übliche Weise, z.B. mittels eines hier nicht dargestellten Zyklons, noch vor einem mit 34 bezeichneten Sauggebläse aus dem Gasstrom abgetrennt werden.

Es werden nun einige Beispiele von Anwendungen des erfindungsgemäßen Verfahrens angegeben, die teils unter Benutzung der zuvor erläuterten Apparatur entstanden.

### Beispiel 1:

In dem Autoklav 10 mit einem Volumen von 5 l wurden 3 kg eines wasser- und aromaarmen, homogenisierten flüssigen Farbkonzentrates (Farbzahl 130.000) vorgelegt, das aus Paprika durch Extraktion mit Hexan gewonnen wurde. Sodann wurde die Flüssigkeit für 90 min von unten nach oben mit Kohlendioxid bei einem Druck von 125 bar und einer Temperatur von 32 °C durchströmt, um das flüssige Farbkonzentrat bei den angegebenen Bedingungen zumindest annähernd mit dem Gas zu sättigen.

Anschließend wurde die Kohlendioxidzufuhr unterbrochen, die Sprühleitung 12 geöffnet und die entstandene gashaltige Lösung durch eine in den Deckel des Sprühturms 24 integrierte Düse 20 mit einem Mündungsdurchmesser von 0,3 mm entspannt. Durch Zudosieren von zusätzlichem Kohlendioxid zur gashaltigen Lösung kurz vor der Entspannungsstelle wurde die Temperatur im Sprühturm auf -25 °C eingestellt. In den Ringraum (s. Figur) um die Düse 20 wurde während des Sprühvorgangs hochdisperses Siliciumdioxid dosiert. Nach einer Sprühzeit von 1 min wurden aus dem Sprühturm 100 g eines homogenen Pulvers entnommen. Der Hilfsstoffanteil (Siliciumdioxid) betrug 30 Gew.%, der Wirkstoffanteil (Farbkonzentrat) somit 70 Gew.%. Die Pulverform des entstandenen Produktes blieb auch bei Erwärmung auf eine Temperatur von 35 °C erhalten. Dies ist insofern bemerkenswert, als daß das Ausgangs-Farbkonzentrat bei einer solchen Temperatur flüssig ist und auch bei Abkühlung auf -18 °C immer noch eine cremige Konsistenz hat. Durch das beschriebene Verfahren gelingt demnach die Stabilisierung der Pulverform bei Temperaturen, die mehr als 50 °C über der Erstarrungstemperatur der im vorliegenden Beispiel zu pulverisierenden Substanz liegen.

### Beispiel 2:

Durch das im Beispiel 1 beschriebene Farbkonzentrat aus Paprika wurde für 3 Stunden Kohlendioxid bei 250 bar und 50 °C strömen gelassen. Die entstandene gashaltige Lösung wurde wie im Beispiel 1 verdüst. Durch Kohlendioxidzufuhr kurz vor der Entspannungsstelle wurde die Temperatur im Sprühturm auf -30 °C eingestellt. Als Hilfsstoff wurde pulverförmiges Polyethylenglykol wie im Beispiel 1 erläutert zugegeben. Nach Abschluß des Versuches konnten 200 g Pulver mit einem Hilfsstoffanteil (Polyethylenglykol) von 70 Gew.% aus dem Sprühturm entnommen werden. Die Pulverform blieb auch bei Erwärmung auf 25 °C erhalten.

### Beispiel 3:

In dem Autoklav 10 mit einem Volumen von 5 l wurden 3 kg eines wasser- und aromaarmen, homogenisierten Farbkonzentrates (Farbzahl 80.000), das aus Paprika durch Extraktion mit Kohlendioxid gewonnen wurde, eingesetzt. Die Flüssigkeit wurde mit Kohlendioxid von unten nach oben bei einem Druck von 125 bar und einer Temperatur von 32 °C für 90 min durchströmt. Anschließend wurde die Kohlendioxidzufuhr unterbrochen und die Sprühleitung geöffnet.

Die gashaltige Lösung wurde mittels einer in den Deckel des Sprühturms 24 integrierten Düse 20 mit einem Mündungsdurchmesser von 0,5 mm entspannt. Bei Entspannung der gasgesättigten Lösung stellte sich ein Sprühturmtemperatur von 0 °C ein. Im Ringraum (s. Figur) um die Düse 20 wurde während des Sprühvorgangs hochdisperses Siliciumdioxid dosiert. Nach einer Sprühzeit von 1 min wurden aus dem Sprühturm 900 g eines homogenen Pulvers entnommen. Der Siliciumdioxidanteil betrug 35 Gew.%. Die Pulverform blieb auch bei Erwärmung auf eine Temperatur von 35 °C erhalten.

### Beispiel 4:

Es wurden 42 kg eines handelsüblichen Paprikaextrakts (Farbzahl 130000), der durch Extraktion von Paprikapulver mit Hexan gewonnen, in einem Autoklav mit einem Volumen von 400 l vorgelegt. Im Extrakt wurde Kohlendioxid bei einem Druck von 250 bar und einer Temperatur von 50 °C gelöst. Anschließend wurde das Farbkonzentrat über eine Düse mit einem Mündungsdurchmesser von 0,8 mm versprüht. Während des Sprühvorgangs wurde mittels einer mit Preßluft angetriebenen Doppelmembranpumpe hochdisperses Siliciumdioxid zudosiert. Die Hilfsstoffzugabe erfolgte punktförmig im Bereich der Düsenmündung.

In 38 min wurden bei einer Temperatur im Sprühturm von -10 °C 55,3 kg Pulver mit einem Anteil an hochdispersem Siliciumdioxid von ca. 24 Gew.% hergestellt. Das Pulver blieb auch bei Raumtemperatur frei fließend.

### Beispiel 5:

In einem Autoklav mit einem Volumen von 1 l wurden 300 g eines Aromaöls, das aus Rosmarin gewonnen wurde, vorgelegt. In der Flüssigkeit wurde bei einem Druck von 100 bar und Raumtemperatur Kohlendioxid aufgelöst. Die gashaltige Lösung wurde über eine Düse mit einem Mündungsdurchmesser von 0,3 mm in einen Sprühturm entspannt. Die Temperatur im Sprühturm betrug -5 °C. Zu dem Freistrahl wurde als Hilfsstoff hochdisperses Siliciumdioxid zugegeben. Es wird ein pulverförmiger Feststoff mit einem Hilfsstoffanteil (Siliciumdioxid) von 25 Gew.% erhalten. Der Wirkstoffanteil (Rosmarin) des erhaltenen Pulvers betrug somit 75 Gew.%.

### Beispiel 6:

In einem Autoklav mit einem Volumen von 1 l wurden 250 g eines Aromaöls, das aus Rosmarin gewonnen wurde, vorgelegt. In der Flüssigkeit wurde bei einem Druck von 150 bar und einer Temperatur von 33 °C Kohlendioxid aufgelöst. Die gashaltige Lösung wurde über eine auf 60 °C temperierte Sprühleitung einer Düse mit einem Mündungsdurchmesser von 0,3 mm zugeführt und in einen Sprühturm entspannt. Durch Dosierung zusätzlichen Kohlendioxids wurde im Sprühturm eine Temperatur von -12 °C eingestellt. Während der Entspannung wurde Palatinit (Isomalt) zugegeben. Es wird ein pulverförmiges Produkt mit einem Anteil an Palatinit von 87 Gew.% erhalten.

### Beispiel 7:

In einem Autoklav mit einem Volumen von 1 l wurden 300 g eines Aromaöls, das aus Rosmarinextrakt gewonnen wurde, vorgelegt. In der Flüssigkeit wurde bei einem Druck von 150 bar und einer Temperatur von 19 °C Kohlendioxid aufgelöst. Die gashaltige Lösung wurde über eine Sprühleitung gleicher Temperatur einer Düse mit einem Mündungsdurchmesser von 0,3 mm zugeführt und in einen Sprühturm entspannt. Durch Dosierung zusätzlichen Kohlendioxids wurde im Sprühturm eine Temperatur von -18 °C eingestellt. Während der Entspannung wurde eine Mischung aus 80 Gew.% Palatinit (Isomalt) und 20 Gew.% hochdispersem Siliciumdioxid zugegeben. Es wird ein pulverförmiges Produkt mit einem Anteil an Hilfsstoff (Palatinit + Siliciumdioxid) von 50 Gew.% erhalten.

### Beispiel 8:

In einem Autoklav mit einem Volumen von 1 l wurden 280 g einer flüssigen Zubereitung eines Pfefferextraktes vorgelegt. Die Zubereitung hat einen Piperingehalt von 40 Gew.%, einen Anteil an Aromaöl von 10 Gew.% und einen Emulgatorgehalt von 15 Gew.%. In der Flüssigkeit wurde bei einem Druck von 70 bar und einer Temperatur von 42 °C Kohlendioxid aufgelöst. Die gashaltige Lösung wurde über eine Sprühleitung gleicher Temperatur einer Düse mit einem Mündungsdurchmesser von 0,3 mm zugeführt und in einen Sprühturm entspannt. Durch Dosierung zusätzlichen Kohlendioxids wurde im Sprühturm eine Temperatur von -14 °C eingestellt. Während der Entspannung wurde hochdisperses Siliciumdioxid zugegeben. Es wird ein pulverförmiges, rieselfähiges Produkt mit einem Anteil an Hilfsstoff (Siliciumdioxid) von 39 Gew.% erhalten.

### Beispiel 9:

In einem Autoklav mit einem Volumen von 1 l wurden 270 g einer flüssigen Zubereitung eines Pfefferextraktes vorgelegt. Die Zubereitung hat eine Piperingehalt von 40 Gew.%, einen Anteil an Aromaöl von unter 5 Gew.% und einen Emulgatorgehalt von 15 Gew.%. In der Flüssigkeit wurde bei einem Druck von 110 bar und einer Temperatur von 42 °C Kohlendioxid aufgelöst. Die gashaltige Lösung wurde über eine Sprühleitung gleicher Temperatur einer Düse mit einem Mündungsdurchmesser von 0,3 mm zugeführt und in einen Sprühturm entspannt. Durch Dosierung zusätzlichen Kohlendioxids wurde im Sprühturm eine Temperatur von -4 °C eingestellt. Während der Entspannung wurde hochdisperses Siliciumdioxid zugegeben. Es wird ein pulverförmiges, rieselfähiges Produkt mit einem Anteil an Hilfsstoff (Siliciumdioxid) von 21 Gew.% erhalten.

### Beispiel 10:

In einem Autoklav mit einem Volumen von 1 l wurden 290 g eines bei Raumtemperatur dünnflüssigen Extraktes aus Sellerie vorgelegt. In der Flüssigkeit wurde bei einem Druck von 160 bar und einer Temperatur von 42 °C Kohlendioxid aufgelöst. Die gashaltige Lösung wurde über eine Sprühleitung gleicher Temperatur einer Düse mit einem Mündungsdurchmesser von 0,3 mm zugeführt und in einen Sprühturm entspannt. Durch Dosierung zusätzlichen Kohlendioxids wurde im Sprühturm eine Temperatur von 5 °C eingestellt. Während der Entspannung wurde hochdisperses Siliciumdioxid zugegeben. Es wird ein pulverförmiges, rieselfähiges Produkt mit einem Anteil an Hilfsstoff (Siliciumdioxid) von 35 Gew.% erhalten.

## Patentansprüche

1. Verfahren zur Herstellung eines pulverförmigen Produktes aus einem **bei Raumtemperatur** flüssigen Stoff oder Stoffgemisch, mit den Schritten:
- Bereitstellen des zu pulverisierenden, flüssigen Stoffes oder Stoffgemisches in einem Druckbehälter,
- Auflösen eines Gases in dem flüssigen Stoff oder Stoffgemisch unter erhöhtem Druck,
- Herausführen der Flüssigkeits/Gas-Lösung aus dem Druckbehälter zu einem Entspannungsorgan, und
- Leiten der Flüssigkeits/Gas-Lösung durch das Entspannungsorgan zur schnellen Entspannung der Lösung,
**dadurch gekennzeichnet**, daß der Lösung bzw. dem Stoff oder Stoffgemisch vor dem Entspannungsorgan, in dem Entspannungsorgan oder nach, insbesondere kurz nach dem Entspannungsorgan ein fester, pulverförmiger Hilfsstoff zugemischt wird.

2. Verfahren nach Anspruch 1,
**dadurch gekennzeichnet**, daß der beim Leiten der Flüssigkeits/Gas-Lösung durch das Entspannungsorgan stattfindende Entspannungsvorgang so durchgeführt wird, daß die Erstarrungstemperatur des Stoffes oder Stoffgemisches in etwa erreicht oder unterschritten wird.

3. Verfahren nach Anspruch 1 oder 2,
**dadurch gekennzeichnet**, daß Gas in dem flüssigen Stoff oder Stoffgemisch solange aufgelöst wird, bis der flüssige Stoff oder das Stoffgemisch im wesentlichen mit dem Gas gesättigt ist.

4. Verfahren nach einem der Ansprüche 1 bis 3,
**dadurch gekennzeichnet**, daß als Gas Kohlendioxid, ein Kohlenwasserstoff, insbesondere Methan, Ethan, Propan, Butan, Ethen, Propen oder ein halogenierter Kohlenwasserstoff, ein Ether, ein Inertgas, insbesondere Stickstoff, Helium oder Argon, ein gasförmiges Oxid, insbesondere Distickstoffoxid oder Schwefeldioxid, Ammoniak oder eine Mischung aus zwei oder mehreren der vorgenannten Gase verwendet wird.

5. Verfahren nach einem der vorhergehenden Ansprüche,
**dadurch gekennzeichnet**, daß der erhöhte Druck, unter dem das Gas in dem flüssigen Stoff oder Stoffgemisch aufgelöst wird, im Bereich von 5 bar bis 800 bar, vorzugsweise im Bereich von 10 bar bis 350 bar, und besonders bevorzugt im Bereich von 20 bar bis 2.50 bar liegt.

6. Verfahren nach einem der vorhergehenden Ansprüche,
**dadurch gekennzeichnet**, daß das Auflösen des Gases in dem. flüssigen Stoff oder Stoffgemisch durch Vermischen des Gases mit dem flüssigen Stoff oder Stoffgemisch beschleunigt wird.

7. Verfahren nach Anspruch 6,
**dadurch gekennzeichnet**, daß das Vermischen des Gases mit dem flüssigen Stoff oder Stoffgemisch mittels eines statischen Mischers, durch Schütteln oder Rollen des Druckbehälters, durch Rühren der im Druckbehälter entstehenden Lösung, durch Rezirkulieren der im Druckbehälter enthaltenen Flüssigphase und/oder Gasphase, oder durch eine Kombination zweier oder mehrerer der vorgenannten Verfahrensweisen erreicht wird.

8. Verfahren nach einem der vorhergehenden Ansprüche,
**dadurch gekennzeichnet**, daß die Partikelgröße des pulverförmigen Hilfsstoffes kleiner als 100 *µ*m, vorzugsweise kleiner als 50 *µ*m ist.

9. Verfahren nach einem der vorhergehenden Ansprüche,
**dadurch gekennzeichnet**, daß als pulverförmiger Hilfsstoff Stärke, modifizierte Stärke, Kochsalz, Zucker, Eiweiße, Gelatine, Titandioxid, Magnesiumstearat, Polyglykole, hochdisperses Siliciumdioxid, Kieselsäure, Bentonit, Kalk, Glutamat, Emulgatoren, insbesondere Phospholipide oder Partialglyceride, Fette, Cellulose und Cellulosederivate, Polymilchsäure, Wachse, Dextrin, Kaolin, Zeolithe, Verdickungsmittel, insbesondere Alginate oder Pectin, Aktivkohle, feinstvermahlene Pflanzenteile oder eine Mischung aus zwei oder mehreren der vorgenannten Stoffe verwendet wird.

10. Verfahren nach einem der vorhergehenden Ansprüche,
**dadurch gekennzeichnet**, daß die Hilfsstoffkonzentration bezogen auf die Gesamtmenge an flüssigem Stoff oder Stoffgemisch und Hilfsstoff zwischen 1 Gew.% und 90 Gew.%, bevorzugt zwischen 10 Gew.% und 80 Gew.% und besonders bevorzugt zwischen 20 Gew.% und 50 Gew.% beträgt.

11. Verfahren nach einem der vorhergehenden Ansprüche,
**dadurch gekennzeichnet**, daß als Entspannungsorgan eine Düse, ein Diffusor, eine Kapillare, eine Blende, ein Ventil oder eine Kombination der genannten Entspannungsorgane verwendet wird.

12. Verfahren nach einem der vorhergehenden Ansprüche,
**dadurch gekennzeichnet**, daß der Hilfsstoff dem aus dem Entspannungsorgan austretenden Stoffstrom im Bereich der Austrittsstelle zugeführt wird.

13. Verfahren nach einem der vorhergehenden Ansprüche,
**dadurch gekennzeichnet**, daß die Entspannung der Lösung in einen Sprühturm erfolgt.

14. Verfahren nach einem der vorhergehenden Ansprüche,
**dadurch gekennzeichnet**, daß zwischen dem Druckbehälter und dem Entspannungsorgan, insbesondere kurz vor der Entspannungsstelle, in die Flüssigkeits/Gas-Lösung zusätzlich Gas eingespeist wird.

15. Verfahren nach einem der Ansprüche 1 bis 12,
**dadurch gekennzeichnet**, daß in dem Entspannungsorgan mittels einer Zweistoffdüse die Flüssigkeits/Gas-Lösung und zusätzlich zugeführtes Gas gemeinsam miteinander entspannt werden.

16. Verfahren nach einem der Ansprüche 1 bis 12,
**dadurch gekennzeichnet**, daß zusammen mit der Zuführung des festen, pulverförmigen Hilfsstoffes zu der Lösung bzw. zu dem Stoff oder Stoffgemisch auch zusätzliches Gas zugeführt wird.

## Claims

1. Process for producing a pulverulent product from a liquid substance or mixture of substances liquid at room temperature, having the steps:
- providing, in a pressure vessel, the liquid substance or mixture of substances to be pulverized,
- dissolving a gas in the liquid substance or mixture of substances under elevated pressure,
- conducting the liquid/gas solution out of the pressure vessel to an expansion element, and
- passing the liquid/gas solution through the expansion element for rapid expansion of the solution,
**characterized in that** a solid, pulverulent auxiliary is admixed to the solution or the substance or the mixture of substances upstream of the expansion element, in the expansion element or downstream, in particular just downstream, of the expansion element.

2. Process according to Claim 1, **characterized in that** the expansion process taking place during passage of the liquid/gas solution through the expansion element is carried out in such a manner that the temperature roughly attains or falls below the solidification temperature of the substance or mixture of substances.

3. Process according to Claim 1 or 2, **characterized in that** gas is dissolved in the liquid substance or mixture of substances until the liquid substance or the mixture of substances is essentially saturated with the gas.

4. Process according to one of Claims 1 to 3, **characterized in that**, as gas, use is made of carbon dioxide, a hydrocarbon, in particular methane, ethane, propane, butane, ethene, propene, or a halogenated hydrocarbon, an ether, an inert gas, in particular nitrogen, helium or argon, a gaseous oxide, in particular dinitrogen oxide or sulphur dioxide, ammonia, or a mixture of two or more of the abovementioned gases.

5. process according to one of the preceding claims,
**characterized in that** the elevated pressure under which the gas is dissolved in the liquid substance or mixture of substances is in the range from 5 bar to 800 bar, preferably in the range from 10 bar to 350 bar, and particularly preferably in the range from 20 bar to 250 bar.

6. Process according to one of the preceding claims,
**characterized in that** the dissolution of the gas in the liquid substance or mixture of substances is accelerated by mixing the gas with the liquid substance or mixture of substances.

7. Process according to Claim 6, **characterized in that** the gas is mixed with the liquid substance or mixture of substances by a static mixer, by shaking or rolling the pressure vessel, by stirring the solution forming in the pressure vessel, by recirculating the liquid phase and/or gas phase present in the pressure vessel, or by a combination of two or more of the abovementined procedures.

8. Process according to one of the preceding claims,
**characterized in that** the particle size of the pulverulent auxiliary is less than 100 *µ*m, preferably less than 50 *µ*m.

9. Process according to one of the preceding claims,
**characterized in that**, as pulverulent auxiliary, use is made of starch, modified starch, common salt, sugar, proteins, gelatin, titanium dioxide, magnesium stearate, polyglycols, highly disperse silicon dioxide, silicic acid, bentonite, lime, glutamate, emulsifiers, in particular phospho-lipids or partial glycerides, fats, cellulose and cellulose derivatives, polylactic acid, waxes, dextrin, kaolin, zeolites, thickeners, in particular alginates or pectin, activated carbon, very finely ground plant components or a mixture of two or more of the abovementioned substances.

10. Process according to one of the preceding claims,
**characterized in that** the auxiliary concentration, based on the total amount of liquid substance or mixture of substances and auxiliary, is between 1% by weight and 90% by weight, preferably between 10% by weight and 80% by weight, and particularly preferably between 20% by weight and 50% by weight.

11. Process according to one of the preceding claims,
**characterized in that**, as expansion element, use is made of a nozzle, a diffuser, a capillary, an orifice plate, a valve or a combination of the said expansion elements.

12. Process according to one of the preceding claims,
**characterized in that** the auxiliary is fed to the mass stream, which is exiting from the expansion element, in the area of the outlet point.

13. process according to one of the preceding claims,
**characterized in that** the solution is expanded into a spray tower.

14. Process according to one of the preceding claims,
**characterized in that** gas is additionally fed into the liquid/gas solution between the pressure vessel and the expansion element, in particular just upstream of the expansion point.

15. Process according to one of Claims 1 to 12, **characterized in that** the liquid/gas solution and additionally supplied gas are expanded together with one another in the expansion element by means of a two-component nozzle.

16. Process according to one of Claims 1 to 12, **characterized in that** additional gas is also fed together with the feed of the solid pulverulent auxiliary to the solution or to the substance or mixture or substances.

## Revendications

1. Procédé de fabrication d'un produit pulvérulent à partir d'un matériau ou d'un mélange de matériaux liquide à la température ambiante, comprenant les opérations consistant à :
- mettre le matériau ou le mélange de matériaux liquide à pulvériser dans un réservoir sous pression,
- dissoudre un gaz dans le matériau ou le mélange de matériaux liquide sous une pression accrue,
- faire sortir la solution de liquide / gaz du réservoir sous pression vers un dispositif de détente, et
- faire passer la solution de liquide / gaz par le dispositif de détente pour une détente rapide de la solution,
**caractérisé en ce qu**'on ajoute et on mélange à la solution, respectivement au matériau ou au mélange de matériaux un matériau auxiliaire solide pulvérulent avant le dispositif de détente, dans le dispositif de détente ou après, en particulier peu après, le dispositif de détente.

2. Procédé selon la revendication 1,
**caractérisé en ce que** le processus de détente qui a lieu lors du passage de la solution de liquide / gaz par le dispositif de détente est effectué de telle façon que la température de solidification du matériau ou du mélange de matériaux soit sensiblement atteinte ou dépassée dans le sens descendant.

3. Procédé selon la revendication 1 ou 2,
**caractérisé en ce que** gaz est dissous dans le matériau ou le mélange de matériaux liquide jusqu'à ce que le matériau ou mélange de matériaux liquide soit essentiellement saturé du gaz.

4. Procédé selon l'une quelconque des revendications 1 à 3,
**caractérisé en ce que** le gaz est le dioxyde de carbone, un hydrocarbure, en particulier le méthane, l'éthane, le propane, le butane, l'éthylène, le propylène ou un hydrocarbure halogéné, un éther, un gaz inerte, en particulier l'azote, l'hélium ou l'argon, un oxyde gazeux, en particulier le dioxyde d'azote ou le dioxyde de soufre, l'ammoniaque ou un mélange de deux ou de plusieurs des gaz susmentionnés.

5. Procédé selon l'une quelconque des revendications précédentes,
**caractérisé en ce que** la pression accrue sous laquelle le gaz est dissous dans le matériau ou le mélange de matériaux liquide se situe dans la plage de 5 à 800 bars, de préférence dans la plage de 10 à 350 bars, et de façon particulièrement préférée dans la plage de 20 à 250 bars.

6. Procédé selon l'une quelconque des revendications précédentes,
**caractérisé en ce que** la dissolution du gaz dans le matériau ou le mélange de matériaux liquide est accélérée par le brassage du gaz avec le matériau liquide ou le mélange de matériaux liquide.

7. Procédé selon la revendication 6,
**caractérisé en ce que** le brassage du gaz avec le matériau ou le mélange de matériaux liquide est réalisé à l'aide d'un mélangeur statique, en appliquant des secousses au réservoir sous pression ou en le faisant rouler, par agitation de la solution produite dans le réservoir sous pression, par recirculation de la phase liquide et / ou la phase gazeuse contenue dans le réservoir sous pression, ou par une combinaison de deux ou plusieurs procédés susmentionnés.

8. Procédé selon l'une quelconque des revendications précédentes, **caractérisé en ce que** la dimension des particules du matériau auxiliaire pulvérulent est inférieure à 100 µm, de préférence inférieure à 50 µm.

9. Procédé selon l'une quelconque des revendications précédentes,
**caractérisé en ce qu**'on utilise comme matériau auxiliaire pulvérulent l'amidon, l'amidon modifié, le sel de cuisine, le sucre, l'albumine, la gélatine, le dioxyde de titane, le stéarate de magnésium, le polyglycol, le dioxyde de silicium fortement dispersé, l'acide silique, la bentonite, la chaux, le glutamate, des émulsifiants, en particulier des phospholipides ou des glycérides partiels, des matériaux gras, la cellulose et des dérivés de cellulose, des résines polylactiques, des cires, la dextrine, le kaolin, des zéolites, des produits épaississants, en particulier des alginates ou de la pectine, le charbon actif, des parties végétales finement broyées ou un mélange de deux ou de plusieurs des matériaux susmentionnés.

10. Procédé selon l'une quelconque des revendications précédentes,
**caractérisé en ce que** la concentration en matériau auxiliaire par rapport à la quantité totale de matériau ou de mélange de matériaux liquide et de matériau auxiliaire se situe entre 10 % en poids et 90 % en poids, de préférence entre 10 % en poids et 80 % en poids et de façon particulièrement préférée entre 20 % en poids et 50 % en poids.

11. Procédé selon l'une quelconque des revendications précédentes,
**caractérisé en ce qu**'on utilise comme dispositif de détente, un éjecteur, un diffuseur, un capillaire, un diaphragme, une vanne ou une combinaison des dispositifs de détente cités.

12. Procédé selon l'une quelconque des revendications précédentes,
**caractérisé en ce que** le matériau auxiliaire est ajouté au flux de matériau sortant du dispositif de détente dans la zone de l'emplacement de sortie.

13. Procédé selon l'une quelconque des revendications précédentes,
**caractérisé en ce que** la détente de la solution a lieu dans une tour de pulvérisation.

14. Procédé selon l'une quelconque des revendications précédentes,
**caractérisé en ce que** du gaz supplémentaire est introduit dans la solution de liquide / gaz entre le réservoir sous pression et le dispositif de détente, en particulier peu avant le poste de détente.

15. Procédé selon l'une quelconque des revendications 1 à 12,
**caractérisé en ce que** la solution de liquide / gaz et le gaz supplémentaire ajouté sont détendus ensemble dans le dispositif de détente au moyen d'un éjecteur binaire.

16. Procédé selon l'une quelconque des revendications 1 à 12,
**caractérisé en ce que** du gaz supplémentaire est aussi introduit ensemble avec l'introduction du matériau auxiliaire solide, pulvérulent dans la solution respectivement dans le matériau ou mélange de matériaux.
